(19)

Europäisches Patentamt

European Patent Office

Office européen des brevets

(11) **EP 4 763 275 A1**

(12) **EUROPEAN PATENT APPLICATION**

(43) Date of publication:
24.06.2026 Bulletin 2026/26

(21) Application number: 24220678.7

(22) Date of filing: **17.12.2024**

(51) International Patent Classification (IPC):
**A61N 5/10** *(2006.01)*

(52) Cooperative Patent Classification (CPC):
**A61N 5/103; A61N 5/1047**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers International AG**
**6312 Steinhausen (CH)**

(72) Inventors:
- **KUUSELA, Esa**
**02320 Espoo (FI)**
- **SABEL, Martin**
**6332 Hagendorn (CH)**

(74) Representative: **Mathisen & Macara LLP**
**Charta House**
**30-38 Church Street**
**Staines-upon-Thames TW18 4EP (GB)**

(54) **METHOD OF IMPROVING RADIATION TREATMENT PLANS**

(57) The invention relates to a method and apparatus which provides a smoothed radiation treatment plan. A time axis is defined for each control point setting a control point time. The control point time is set to be greater than a minimum time required to move all of the components of a treatment machine from a first control point to a second control point in order to slow down the components of the treatment machine resulting in a less jerky delivery.

```
┌─────────────────────────────────────────────┐
│                    S301                       │
│             Receive treatment plan            │
└─────────────────────────────────────────────┘
                       │
                       ▼
┌─────────────────────────────────────────────┐
│                    S302                       │
│            Determine control points           │
└─────────────────────────────────────────────┘
                       │
                       ▼
┌─────────────────────────────────────────────┐
│                    S303                       │
│  Determine theoretical minimum time for each  │
│                 control point                 │
└─────────────────────────────────────────────┘
                       │
                       ▼
┌─────────────────────────────────────────────┐
│                    S304                       │
│  Determine control point time for each        │
│                control point                  │
└─────────────────────────────────────────────┘
                       │
                       ▼
┌─────────────────────────────────────────────┐
│                    S305                       │
│   Determine trajectories for each control     │
│                   point                       │
└─────────────────────────────────────────────┘
                       │
                       ▼
┌─────────────────────────────────────────────┐
│                    S306                       │
│   Generate smoothed radiation treatment plan  │
└─────────────────────────────────────────────┘
```

FIGURE 3

Processed by Luminess, 75001 PARIS (FR)

**Description**

Technical Field

These teachings relate generally to treating a patient's planning target volume with energy pursuant to an energy-based treatment plan and more particularly to improving an energy-based treatment plan.

Background

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted tumors and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

When a radiation treatment plan is generated in a treatment planning system, the instructions for the radiation therapy treatment machine are given as a set of control points. These control points specify the desired axis positions of components of the treatment machine (gantry angle, collimator angle, leaf positions, jaw positions, cumulative meterset weight, couch positions) in consecutive instances. However, the time (in seconds) required for the treatment machine to move from a first control point to a second control point, and so on, is not specified, only the order (sequence) of the control points is given. A controller of the treatment machine determines the actual timings for the delivery of the treatment plan. Some known treatment machines aim to generate a treatment plan which can be delivered in the shortest possible time (dependent on the speed and acceleration limits of each component). Unfortunately, in order to deliver a treatment plan in the shortest possible time, treatment machines often undergo general accelerations and decelerations induced by slowly accelerating fast axes (especially gantry) which in turn will introduce similar acceleration and deceleration to the couch-degrees-of-freedom, resulting in jerky motions.

Delivering a treatment plan in the shortest possible time has benefits, in that the amount of time a patient needs to hold still during the treatment is minimized. However, in cases of trajectory treatments where the couch is also moving, it would be beneficial to make sure that the couch motion is as smooth as possible so that the patient does not feel unnecessary accelerations or decelerations. This is especially important in longitudinal couch motion, for example when irradiating a whole spine, where a relatively long stable linear motion would feel the same to the patient as not moving at all.

As used herein, "smooth" will be understood to refer to reducing the amount of rapid increase and decreases of speeds of each component of a treatment system.

Summary

According to a first embodiment, there is provided a method of generating a radiation therapy treatment plan. The treatment plan comprises at least two consecutive control points, each control point comprising an axis position for each one or more moveable components of a treatment machine at that control point. The method comprising: determining a theoretical minimum time for each control point, the theoretical minimum time comprising a time for all of the one or more components of the treatment machine to move from their first axis position at a first control point to their second axis position at a consecutive second control point, the theoretical minimum time determined based on a maximum speed each of the one or more components of the treatment machine is capable of moving; determining a control point time for each control point, the control point time comprising a time for all of the one or more components of the treatment machine to move from their first axis positions at the first control point to their second axis positions at the second control point, wherein the control point time is greater than the theoretical minimum time; determining trajectories for each of the one or more components of the treatment machine for each control point, the trajectory for each of the one or more components of the treatment machine comprising a path for the component of the treatment machine to move from their first axis position at the first control point to their second axis position at the second control point within the control point time; and applying the determined trajectories and the determined control point times to each control point to generate the radiation therapy treatment plan.

According to another embodiment, the method further comprises: synchronising the one or more components of the treatment machine to all move from their first axis positions at the first control point to their second axis positions at the second control point and arrive at their second axis positions at the second control point at the control point time.

According to another embodiment, the method further comprises: determining a time greater than the control point time to move all of the one or more components of the treatment machine from their first axis positions at the first control point to their second axis positions at the second control point, when one or more of the components of the treatment machine requires greater than the control point time to move from their first axis positions at the first control point to their second axis positions at the second control point; and applying the determined trajectories and the determined time greater than the control point time to the first control point to generate the radiation therapy treatment plan.

According to another embodiment, the method further comprises: synchronising the one or more components of the treatment machine to all move from their first axis positions at the first control point to their second axis positions at the second control point and arrive at their second axis positions at the second control point at the time greater than the control point time.

According to another embodiment, the method further comprises: determining a control point speed for each of the one or more components of the treatment machine for each control point, wherein the control point speed for each of the one or more components of the treatment machine is determined using the control point time and a distance between the first axis position of the component at the first control point and the second axis positions of the component at the second control point; and applying the control point speed to each of the one or more components of the treatment machine for each control point to generate the radiation therapy treatment plan.

According to another embodiment, the method further comprises: determining a control point speed for each of the one or more components of the treatment machine for each control point, wherein the control point speed for each of the one or more components of the treatment machine is determined using the time greater than the control point time and a distance between the first axis position of the component at the first control point and the second axis positions of the component at the second control point; and applying the control point speed to each of the one or more components of the treatment machine for the first control point to generate the radiation therapy treatment plan.

According to another embodiment, a control point time determined for a second control point is the same as a control point time determined for a first control point; or a control point time determined for a second control point is different from a control point time determined for a first control point.

According to another embodiment, the trajectory for one or more components of the treatment machine comprises no trajectory so that the one or more components of the treatment machine remain at the first axis position at the second control point.

According to another embodiment, the first axis position at the first control point of each of the one or more components may be the same as or different from the first axis position at the first control point of the other one or more components.

According to a second embodiment, there is provided an apparatus to facilitate generation of a radiation treatment plan, the treatment plan comprising at least two consecutive control points, each control point comprising an axis position for each one or more moveable components of a treatment machine at that control point. The apparatus comprising: a control circuit configured to: determine a theoretical minimum time for each control point, the theoretical minimum time comprising a time for all of the one or more components of the treatment machine to move from their first axis position at a first control point to their second axis position at a consecutive second control point, the theoretical minimum time determined based on a maximum speed each of the one or more components of the treatment machine is capable of moving; determine a control point time for each control point, the control point time comprising a time for all of the one or more components of the treatment machine to move from their first axis positions at the first control point to their second axis positions at the second control point, wherein the control point time is greater than the theoretical minimum time; determine trajectories for each of the one or more components of the treatment machine for each control point, the trajectory for each of the one or more components of the treatment machine comprising a path for the component of the treatment machine to move from their first axis position at the first control point to their second axis position at the second control point within the control point time; and apply the determined trajectories and the determined control point times to each control point to generate the radiation therapy treatment plan.

According to another embodiment, the control circuit is further configured to: synchronise the one or more components of the treatment machine to all move from their first axis positions at the first control point to their second axis positions at the second control point and arrive at their second axis positions at the second control point at the control point time.

According to another embodiment, the control circuit is further configured to: determine a time greater than the control point time to move all of the one or more components of the treatment machine from their first axis positions at the first control point to their second axis positions at the second control point, when one or more of the components of the treatment machine requires greater than the control point time to move from their first axis positions at the first control point to their second axis positions at the second control point; and apply the determined trajectories and the determined time greater than the control point time to the first control point to generate the radiation therapy treatment plan.

According to another embodiment, the control circuit is further configured to: synchronise the one or more components of the treatment machine to all move from their first axis positions at the first control point to their second axis positions at the second control point and arrive at their second axis positions at the second control point at the time greater than the control point time.

According to another embodiment, the control circuit is further configured to: determine a control point speed for each of the one or more components of the treatment machine for each control point, wherein the control point speed for each of the one or more components of the treatment machine is determined using the control point time and a distance between the first axis position of the component at the first control point and the second axis positions of the component at the second control point; and apply the determined control point speed to each of the one or more components of the treatment machine for each control point to generate the radiation therapy treatment plan.

According to another embodiment, the control circuit is further configured to: determine a control point speed for each of the one or more components of the treatment machine for each control point, wherein the control point speed for each of the one or more components of the treatment machine is determined using the time greater than the control point time and a distance between the first axis position of the component at the first control point and the second axis positions of the component at the second control point; and apply the determined control point speed to each of the one or more components of the treatment machine for the first control point to generate the radiation therapy treatment plan..

According to a third embodiment, there is provided a method of generating a radiation therapy treatment plan, the treatment plan comprising at least two consecutive control points, each control point comprising an axis position for each one or more moveable components of a treatment machine at that control point. The method comprising: determining a theoretical minimum time for each control point, the theoretical minimum time comprising a time for all of the one or more components of the treatment machine to move from their first axis position at a first control point to their second axis position at a consecutive second control point, the theoretical minimum time determined based on a maximum speed each of the one or more components of the treatment machine is capable of moving; determining a control point time for each control point, the control point time comprising a time for all of the one or more components of the treatment machine to move from their first axis positions at the first control point to their second axis positions at the second control point, wherein the control point time is greater than the theoretical minimum time; determining a control point dose rate for each control point, the control point dose rate comprising a dose rate to be administered by the treatment machine as the one or more components of the treatment machine move from their first axis positions at the first control point to their second axis positions at the second control point, wherein the smoothing dose rate is determined using the control point time and a predetermined amount of units of energy to be administered as the one or more components of the treatment machine move from their first axis positions at the first control point to their second axis positions at the second control point determining trajectories for each of the one or more components of the treatment machine for each control point, the trajectory for each of the one or more components of the treatment machine comprising a path for the component of the treatment machine to move from their first axis position at the first control point to their second axis position at the second control point within the control point time; and applying the determined trajectories and the determined control point dose rates to each control point to generate the radiation therapy treatment plan

According to another embodiment, the method further comprises: determining a control point dose rate for each control point, the control point dose rate comprising a dose rate to be administered by the treatment machine as the one or more components of the treatment machine move from their first axis positions at the first control point to their second axis positions at the second control point, wherein the control point dose rate is determined using the time greater than the control point time and a predetermined amount of units of energy to be administered as the one or more components of the treatment machine move from their first axis positions at the first control point to their second axis positions at the second control point; and applying the control point dose rates to the first control point to generate the radiation therapy treatment plan.

According to a fourth embodiment, there is provided an apparatus to facilitate generation of a radiation treatment plan, the treatment plan comprising at least two consecutive control points, each control point comprising an axis position for each one or more moveable components of a treatment machine at that control point. The apparatus comprises a control circuit configured to: determine a theoretical minimum time for each control point, the theoretical minimum time comprising a time for all of the one or more components of the treatment machine to move from their first axis position at a first control point to their second axis position at a consecutive second control point, the theoretical minimum time determined based on a maximum speed each of the one or more components of the treatment machine is capable of moving; determine a control point time for each control point, the control point time comprising a time for all of the one or more components of the treatment machine to move from their first axis positions at the first control point to their second axis positions at the second control point, wherein the control point time is greater than the theoretical minimum time; determine a control point dose rate for each control point, the control point dose rate comprising a dose rate to be administered by the treatment machine as the one or more components of the treatment machine move from their first axis positions at the first control point to their second axis positions at the second control point, wherein the control point dose rate is determined using the control point time and a predetermined amount of units of energy to be administered as the one or more components of the treatment machine move from their first axis positions at the first control point to their second axis positions at the second control point; determine trajectories for each of the one or more components of the treatment machine for each control point, the trajectory for each of the one or more components of the treatment machine comprising a path for the component of the treatment machine to move from their first axis position at the first control point to their second axis position at the second control point within the control point time; and apply the determined trajectories and the determined control point dose rate to each control point to generate the radiation therapy treatment plan.

According to another embodiment, the control circuit is further configured to: determine a control point dose rate for each control point, the control point dose rate comprising a dose rate to be administered by the treatment machine as the one or more components of the treatment machine move from their first axis positions at the first control point to their second axis positions at the second control point, wherein the control point dose rate is determined using the time greater than the control point time and a predetermined amount of units of energy to be administered as the one or more components of the treatment machine move from their first axis positions at the first control point to their second axis positions at the second control point; and apply the control point dose rates to the first control point to generate the radiation therapy treatment plan.

Brief Description of the Drawings

The above needs are at least partially met through provision of methods for improving radiation treatment plans described in the following detailed description, particularly when studied in conjunction with the Figures, wherein:

Figure 1 comprises a block diagram of a radiation therapy treatment system;

Figure 2 comprises a simplified illustration of movements of a rotating gantry and a patient support couch;

Figure 3 comprises a flow diagram illustrating a method for generating an energy-based treatment plan;

Figures 4A-4E illustrate schematically simplified axes of a treatment plan;

Figure 5 comprises a flow diagram illustrating another method for generating an energy-based treatment plan; and

Figure 6 comprises a flow diagram illustrating another method for generating an energy-based treatment plan.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

Detailed Description

Generally speaking, pursuant to these various embodiments, a method and apparatus is disclosed for providing a smoothed radiation treatment plan. A time axis is defined for each control point, the time axis being set at a time greater than a minimum time required to move all of the components of a treatment machine during a control point interval to slow down the components of the treatment machine and create a smoother delivery.

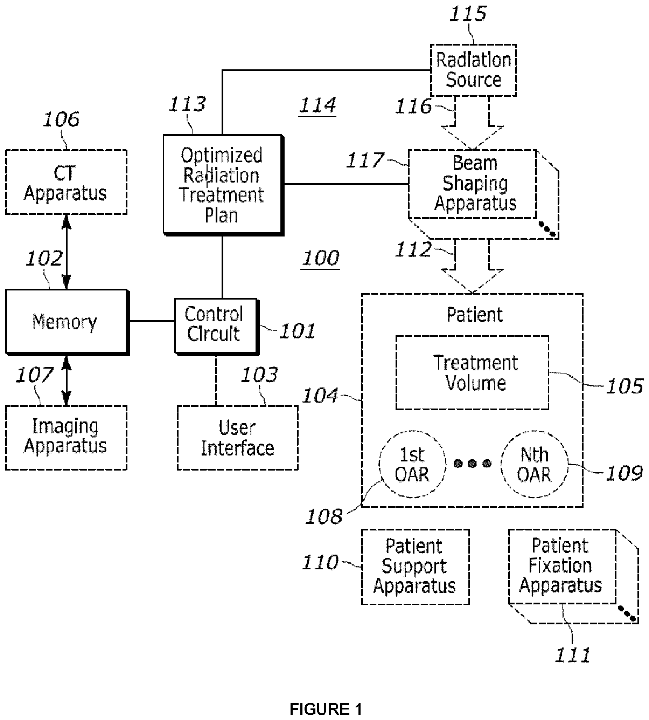

These and other benefits may become clearer upon making a thorough review and study of the following detailed description. Referring now to the drawings, and in particular to Figure 1, an illustrative apparatus 100 that is compatible with many of these teachings will first be described.

Figure 1 illustrates schematically an example of a radiation therapy treatment system (RT system) 100. The RT system 100 comprises a control circuit 101. Being a "circuit," the control circuit 101 therefore comprises structure that includes at least one, and typically many, electrically-conductive paths (such as paths comprised of a conductive metal such as copper or silver) that convey electricity in an ordered manner. The electrically-conductive paths will also typically include corresponding electrical components, both passive (such as resistors and capacitors) and active (such as any of a variety of semiconductor-based devices) as appropriate, to permit the circuit to effect the control aspect of these teachings.

A control circuit 101 can comprise a fixed-purpose hard-wired hardware platform, including but not limited to an application-specific integrated circuit (ASIC), which is an integrated circuit that is customized by design for a particular use, rather than intended for general-purpose use, a field-programmable gate array (FPGA), and the like, or can comprise a partially or wholly-programmable hardware platform, including but not limited to microcontrollers, microprocessors, and the like. These architectural options for such structures are well known and understood in the art and require no further description here. The control circuit 101 is configured, for example, by using corresponding programming as will be well understood by those skilled in the art, to carry out one or more of the steps, actions, and/or functions described herein.

The control circuit 101 operably couples to a memory 102. The memory 102 may be integral to the control circuit 101 or can be physically discrete, in whole or in part, from the control circuit 101 as desired. The memory 102 can also be local with respect to the control circuit 101, where, for example, both share a common circuit board, chassis, power supply, and/or housing, or can be partially or wholly remote with respect to the control circuit 101, where, for example, the memory 102 is physically located in another facility, area, or country as compared to the control circuit 101.

In addition to information such as information regarding a particular patient and information regarding a particular radiation treatment system as described herein, the memory 102 can serve, for example, to non-transitorily store the computer instructions that, when executed by the control circuit 101, cause the control circuit 101 to behave as described herein. As used herein, this reference to "non-transitorily" will be understood to refer to a non-ephemeral state for the stored contents and hence excludes when the stored contents merely constitute signals or waves rather than volatility of the storage media itself and hence includes both non-volatile memory, such as read-only memory (ROM) as well as volatile memory, such as a dynamic random access memory (DRAM).

The control circuit 101 may be operably coupled to a user interface 103. The user interface 103 may comprise any of a variety of user-input mechanisms, such as, but not limited to, keyboards and keypads, cursor-control devices, touch-sensitive displays, speech-recognition interfaces, gesture-recognition interfaces, and so forth, and/or user-output mechanisms, such as, but not limited to, visual displays, audio transducers, printers, and so forth, to facilitate receiving information and/or instructions from a user and/or providing information to a user.

The control circuit 101 may also be operably coupled to a network interface (not shown) to communicate with other elements, both within the apparatus 100 and external thereto, via the network interface. Network interfaces, including both wireless and non-wireless platforms, are well understood in the art and require no elaboration here.

A computed tomography apparatus 106 and/or other imaging apparatus 107 as known in the art may source some or all of any desired patient-related imaging information.

The control circuit 101 is operably coupled to an energy-based treatment platform 114 that is configured to deliver therapeutic energy 112 to a patient 104 having at least one treatment volume 105 and one or more organs-at-risk (OAR), represented in Figure 1 by a first through an Nth organ-at-risk 108 and 109, in accordance with energy-based treatment plan 113. These teachings are generally applicable for use with any of a wide variety of energy-based treatment platforms/apparatuses. Typically, the energy-based treatment platform 114 will include an energy source such as a radiation source 115 of ionizing radiation 116.

The radiation source 115 may be configured to generate a proton beam, electron beam, or neutron beam, as a form of radiation for treatment purposes.

The radiation source 115 may be selectively moved via a gantry along an arcuate pathway, where the pathway encompasses, at least to some extent, the patient themselves during administration of the treatment. The arcuate pathway may comprise a complete or nearly complete circle as desired. The control circuit 101 controls the movement of the radiation source 115 along that arcuate pathway, and controls when the radiation source 115 starts moving, stops moving, accelerates, decelerates, and a velocity at which the radiation source 115 travels along the arcuate pathway.

Alternatively, radiation source 115 may be selectively moved via an arm gantry along a straight pathway, in any direction (i.e., any X, Y, or Z direction) where the pathway encompasses, at least to some extent, the patient themselves during administration of the treatment. The control circuit 101 controls the movement of the radiation source 115 along the straight pathway, and controls when the radiation source 115 starts moving, stops moving, accelerates, decelerates, and a velocity at which the radiation source 115 travels along the straight pathway.

The radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., x-rays) 116 and high energy electrons.

The energy-based treatment platform 114 may also comprise one or more support apparatuses 110 (such as a couch) to support the patient 104 during the treatment session, and one or more patient fixation apparatuses 111.

The energy-based treatment platform 114, may also comprise one or more energy-shaping apparatuses (for example, beam-shaping apparatuses 117 such as jaws, multileaf collimators (MLC), and so forth) to provide selective energy shaping and/or energy modulation as desired. The control circuit 101 controls the movement of the beam-shaping apparatuses 117 when the beam-shaping apparatuses 117 starts moving, stops moving, accelerates, decelerates, and a velocity at which the beam-shaping apparatuses 117 moves.

The patient support apparatus 110 is selectively controllable to move in any direction (i.e., X, Y, or Z direction or yaw) during an energy-based treatment session by the control circuit 101. The control circuit 101 controls the direction of movement of the patient support apparatus 110, when the patient support apparatus 110 starts moving, stops moving, accelerates, decelerates, and a velocity at which the patient support apparatus 110 moves.

As the foregoing elements and systems are well understood in the art, further elaboration in these regards is not provided here except where otherwise relevant to the description.

The control circuit 101 generates the radiation therapy treatment plan instructions for the radiation therapy treatment system as a set of sequential control points. Each control point specifies the required axis positions of the components of the radiation therapy treatment system at that control point, for example, the gantry angle, the collimator angle, the leaf positions, the jaw positions, the couch position etc., as well as specifying cumulative meterset weight. The couch axis position may be defined as X, Y, and Z coordinates and yaw. The gantry angle axis position and collimator angle axis position may be defined as one angular coordinate each. The leaf axis positions and the jaw axis positions have one coordinate describing how much they are retracted from or extended over the center line. The cumulative meterset weight indicates what portion of the total Monitor Units (MU's) to be delivered have been delivered when the control point is reached. A control point is defined for every instance where one or more of the component axis positions changes.

Each component of the radiation therapy treatment system is capable of movement at speeds up to its maximum speed and is capable of accelerations/decelerations up to its maximum acceleration rate/maximum deceleration rate. Each component of the radiation therapy treatment system may have a different maximum speed from the maximum speeds of the other components of the radiation therapy treatment system. In addition, each component of the radiation therapy treatment system may have a different maximum acceleration rate/maximum deceleration rate from the maximum acceleration rates/maximum deceleration rates of the other components of the radiation therapy treatment system.

The control circuit 101 determines a trajectory for each component of the radiation therapy treatment system to follow in order to move that component from a first axis position defined in a first control point to a second axis position defined in a second control point. In addition, the control circuit 101 determines a velocity for each component of the radiation therapy treatment system to move at whilst following the trajectory from the first axis position to the second axis position.

The components of the radiation therapy treatment system are required to be synchronised so that they all leave the first control point at the same first instance and all arrive at the second control point at the same second instance. As stated above, each component of the radiation therapy treatment system is capable of movement at speeds up to its maximum speed and accelerate/decelerate at rates up to its maximum acceleration rate/maximum deceleration rate, and each component may have a its own maximum speed and maximum acceleration rate/maximum deceleration rate.

In order to optimize treatment time and complete treatment plans as quickly as possible (dependent on the speed and acceleration limits of each component), conventional control circuits determine how quickly each component of the system can move from its first position at a first control point to its required second position at a second control point. How quickly each component can move is determined based on the distance required to be traveled by each component between the first and second control points and the known maximum speeds and acceleration/deceleration rates of each component. Since all of the components are required to arrive at the second control point at the same time, the time required for all of the components to move between the first and second control points will be determined by the slowest component for that control point interval. For example, a rotating gantry is capable of moving from its required position at a first control point to its required position at a second control point in time interval x, by moving at its maximum speed and maximum acceleration/deceleration rates, and a patient support couch is capable of moving from its required position at the first control point to its required position at the second control point in time interval y, by moving at its maximum speed and maximum acceleration/deceleration rates. The time interval x is less than time interval y. Since the rotating gantry and patient support couch are required to arrive at their own required positions at the second control point at the same time, the rotating gantry is slowed down so that it also takes time interval y to arrive at the second control point, it moves at a speed less than its maximum and/or accelerates/decelerates at a rate(s) less than its maximum acceleration/deceleration rates .

Each interval between different control points will have a different time interval determined based on the which components are required to move during the control point interval, the distances required to be traveled by each component in the control point interval and the maximum speeds and acceleration/deceleration rates of each component moving during the control point interval.

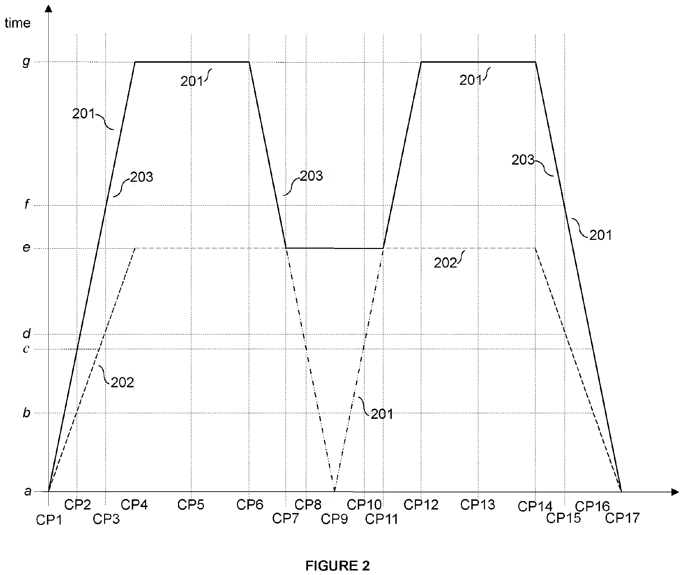

Figure 2 is a simplified illustration of the movements of a rotating gantry (long dash dot dot line 201) and a patient support couch (dash line 202) when both move at their maximum speeds and acceleration/deceleration rates. Figure 2 also illustrates the movements of the rotating gantry and the patient support couch when they have been synchronised to arrive at the control points at the same time (solid line 203). The y-axis of Figure 2 represents time, i.e., it indicates the time it takes for each of the rotating gantry and the patient support couch to move from its axis position described in the previous control point to its axis position described the next sequential control point. It is important to note that the y-axis of Figure 2 is not a cumulative time axis and therefore, it does not represent the total time taken. The x-axis of Figure 2 represents the control points. In reality, the x axis illustrated in Figure 2 comprises more control points than it is possible to illustrate separately. For example, the movements illustrated in Figure 2 may comprise 100 or more control points. However, for simplicity, only a few control points are illustrated.

As illustrated in Figure 2, both the rotating gantry and the couch can move at different rates from each other. Movement of the couch, when moving at it maximum speeds and acceleration/deceleration rates is illustrated by dash line 202. Figure 2 illustrates the movement of the couch from CP1 to CP2 requiring a time interval of b, movement of the couch from CP2 to CP3 requiring a time interval of d, and movement of the couch from CP3 to CP4 requiring a time interval of e. The couch then moves from CP4 to CP5, CP5 to CP6, CP6 to CP7, CP7 to CP8, CP8 to CP9, CP9 to CP10, CP10 to CP11, CP11 to CP12, CP12 to CP13 and CP13 to CP14. Each movement of the couch between CP4 to C14 requires the same time interval of e. Finally, the couch requires a time interval of d to move from CP14 to CP15, a time interval of b to move from CP15 to CP16 and a time interval of a to move from CP16 to CP17.

Movement of the gantry, when moving at it maximum speeds and acceleration/deceleration rates is illustrated by dash dot dot line 201. Figure 2 illustrates the movement of the gantry from CP1 to CP2 requiring a time interval of c, movement of the gantry from CP2 to CP3 requiring a time interval of f, and movement of the gantry from CP3 to CP4 requiring a time interval of g. The gantry then moves from CP4 to CP5 and CP5 to CP6. Each movement of the gantry between CP4 to C6 requires the same time interval of g. A rotating gantry follows an arcuate pathway and decelerate as it reaches the end of its arc before changing direction, to reverse back along the arc. This is illustrated in Figure 2 as the gantry moves from CP6 to CP9 to CP12. The gantry requires a time interval of e to move from CP6 to CP7, a time interval of c to move from CP7 to CP8, a time interval of a to move from CP8 to CP9, a time interval of c to move from CP9 to CP10, a time interval of e to move from CP10 to CP11 and a time interval of g to move from CP11 to CP12. The gantry then moves from CP12 to CP13 and CP13 to CP14. Each movement of the gantry between CP12 to C14 requires the same time interval of g. Finally, the gantry requires a time interval of f to move from CP14 to CP15, a time interval of c to move from CP15 to CP16 and a time interval of a to move from CP16 to CP17.

In the example illustrated Figure 2, the rotating gantry (long dash dot dot line 201) and the patient support couch (dash line 202) are arriving at the control points at different times, which is not desirable. Therefore, a synchronised movement of the gantry and couch is illustrated in Figure 2 by the solid line 203. The solid line 203 illustrates how much time each control point actually takes when only one component (the slowest component) is moving with full speed, the other components being slowed down so that there is synchronised movement.

As illustrated by the solid line 203, movement of the couch is slowed down between CP1 to CP2, so that it also takes time interval c to move from CP1 to CP2. The same applies from CP2 to CP7, the couch is slowed down so that it arrives at the control points at the same time at the gantry. However, at CP7 to CP11 movement of the gantry requires less time than movement of the couch, such that the movement of the gantry is slowed down to arrives at CP7 to CP11 at the same time at the couch. The solid line 203 illustrated in Figure 2 is a simplified example of a theoretical minimum time described in further detail below.

IIn order to optimize the treatment time and complete the treatment plan as quickly as possible (dependent on the speed and acceleration limits of each component), conventional control circuits move the components at their fastest possible speed and the components undergo general accelerations and decelerations induced by slowly accelerating fast axes (for example, the gantry) which in turn will introduce similar accelerations and decelerations to the couch-degrees-of-freedom and other components. In the example of Figure 2, the maximum speed at which the system can run at is initially the gantry speed, as it requires the most time to move between control points. When the gantry requires less time to move between control points, the maximum speed at which the system can run is the couch speed. However, this can result in jerky motion due to constant changes in what is considered the maximum speed.

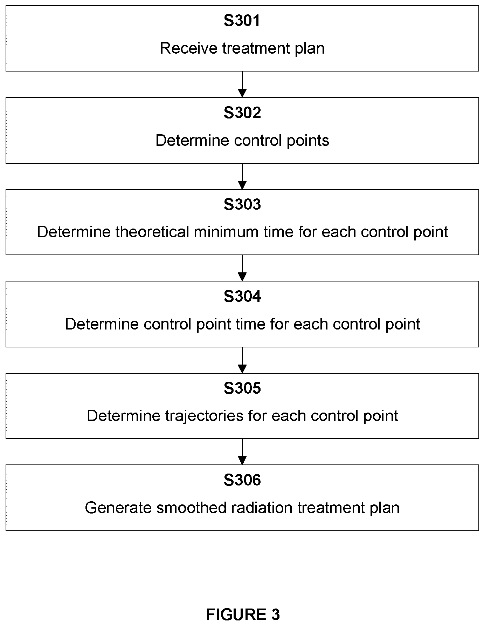

Referring now to Figure 3, a method that can be carried out, for example, in conjunction with the above-described radiation therapy treatment machine (and more particularly via the aforementioned control circuit 101) will be described. Generally speaking, the method serves to facilitate generating a smoothed radiation treatment plan to thereby facilitate treating a particular patient with therapeutic radiation in accordance with the smoothed radiation treatment plan.

In this illustrative example the control circuit 101 is configured to ultimately output a smoothed energy-based treatment plan (such as, for example, a smoothed radiation treatment plan). This energy-based treatment plan typically comprises specified time axis values as well as the specified position axis values for each control point of a plurality of sequential consecutive control points. The control circuit then generates the smoothed treatment plan.

The specified time axis value defines how long the components of the treatment machine shall take to move from a first control point to a next sequential second control point. Typically, a theoretical minimum time is needed for the required components of the treatment machine to move from a first control point to a second control point, the theoretical minimum time being determined based on the known maximum speeds and acceleration/deceleration rates of the components required to move in the control point interval. The specified time axis value is determined to be greater than the theoretical minimum time, thereby essentially slowing down the treatment plan to create a smoother delivery. The specified time axis value may be defined as time in seconds, clock pulses, or other convenient units.

In a simplified example, a treatment machine may comprise two components, a gantry and a couch. The couch is capable of moving at a maximum speed of a m/s and the gantry is capable of moving at a maximum speed of b m/s, b being greater/faster than a. If both components are required to move from the same first control point CP1 to the same second control point CP2 and both components are capable of moving at their maximum speeds, then the theoretical minimum time will be time the required to move the slower of the components, in this example, the couch, from CP1 to CP2. In reality, treatment machines are more complicated than this as they comprise more than two moveable components, each component may be at a different location from each other at CP1 and CP2 so that each component may have a different distance to travel between control points. In addition, each component may have different acceleration/deceleration rates from each other. Furthermore, in order to avoid clashes of components, each component may not be able to travel at its maximum speed for the entirety of the control point.

Although treatment machines comprise multiple moveable components, often only a few of those components will have dominant effect on the time required to deliver the treatment plan.

Referring now to Figure 3, a method for generating an energy-based treatment plan that can be carried out by the aforementioned control circuit 101 will be described. A predetermined patient treatment plan (which has not been processed to optimize timings) is received at a treatment system at step S301.

A treatment planning system determines a plurality of control points for the administration of the treatment plan to a patient at step S302. According to one embodiment, a treatment planning system, which may be a separate workstation, generates the treatment plan, rather than the treatment machine. The plurality of control points comprises at least two consecutive control points. A control point is defined for every instance where one or more of the component axis positions changes.

According to one embodiment, the treatment system determines the control points relatively evenly in time, taking into account the set up and capabilities of the components of the treatment machine. For example, the control points need to be spaced realistically in time, i.e., there must be enough time between the control points for the components of the treatment system which are required to move, to move from the first control point to the second control point etc.

Each control point of the treatment plan defines a machine axis for each of the one or more moveable components of the treatment machine at that control point. A 'machine axis' refers to any independently moving part of the treatment machine. The gantry is one (rotational) axis, the collimator is another one. Each leaf and jaw is considered to be one (linear) axis since it can move independently forward (to block the beam or part of it) or backward (to reveal some parts of the beam).

During application of the treatment plan, the moveable components of a treatment machine are required to move from their defined axis position at the first control point to their defined axis position at the next sequential, second, control point. It is possible that one or more of the moveable components of the treatment machine are not required to move between control points, in which case their defined axis position at the second control point will be the same as their defined axis position at the first control point. In addition, the first axis position at the first control point of each of the one or more components may be the same as the first axis position at the first control point of the other one or more components. For example, any of the leaves in one MLC bank can have the same axis position when the leaves are equal distance from the centerline. The leaves have naturally a different location in another dimension, but since they do not move in this direction, it is not considered an axis position. Furthermore, the first axis position at the first control point of each of the one or more components may be different from the first axis position at the first control point of the other one or more components

It is also possible for the components of the treatment machine to be held at a control point for a predetermined period of time. In one embodiment, such an arrangement may be expressed as two consecutive control points, where one or more of the components of the treatment machine do not move between the two control points.

Each moveable component of a treatment machine is capable of moving at a maximum speed and a maximum acceleration/deceleration rate. At step S303, the control circuit 101 of the treatment system determines a theoretical minimum time (first theoretical minimum time) required to move the components of the treatment machine from their defined axis positions at the first control point to their defined axis positions at the next sequential control point, i.e., the second control point. This minimum time is considered to be the theoretical minimum time of the first control point interval. The theoretical minimum time (second theoretical minimum time) required to move the components of the treatment machine from their defined axis positions at the second control point to their defined axis positions at the next sequential third control point, is considered to be the theoretical minimum time of the second control point interval. The theoretical minimum time (third theoretical minimum time) required to move the components of the treatment machine from their defined axis positions at the third control point to their defined axis positions at the next sequential fourth control point, is considered to be the theoretical minimum time of the third control point interval, and so on.

As discussed above, each component of the treatment system may have a maximum speed and a maximum acceleration/deceleration rate which is different from a maximum speed and a maximum acceleration/deceleration rate of the other components of the treatment system. The theoretical minimum time is determined based on the assumption that each component may move at its maximum speed and maximum acceleration/deceleration rate. In addition, each component of the treatment system may have a distance to travel between control points which is different from a distance to travel between control points of the other components of the treatment system.

The theoretical minimum time is an accumulative time, meaning that it is the time required for all of the components of the treatment machine which are required to move, to move, taking into account that the components may move at the same time, for all or part of the time. The components of the treatment system are required to move synchronously between control points, so that all of the components, which are required to move, arrive at the next control point at the same time. The theoretical minimum time is determined as the time required for all of the components to move from a first control point to a second sequential control point and arrive at the second control point at the same time, taking into account the maximum speed, acceleration, deceleration and path required for each component. Accordingly, some of the components may not be moving at their maximum speed/acceleration rate/deceleration rate or may not be moving at their maximum speed/acceleration rate/deceleration rate for the whole time they are moving between control points. However, the theoretical minimum time is the minimum time needed for all of the components to move from the first control point to the second sequential control point as quickly as possible. It should be noted that although it is described here that the components arrive at the control points at the same time, in practice there are small tolerance values that allow the system to optimize the trajectories of individual axis and allows the machine to tolerate small misplacement of individual moving components.

At step S304, a control point time is determined for each control point. The control point time is set to be greater than the theoretical minimum time, so that the control point time essentially slows down the treatment machines application of the treatment plan. The aim of the control point time is to slow down the administration of the treatment plan resulting in a smoother application of the treatment plan and avoiding "jerky" movements. Jerky movements are considered to be movements which include rapid accelerations and decelerations of the same component between two consecutive control points. The control point time introduces a virtual new axis to the treatment machine, a specified time axis, such that each control point defines a position axis and a time axis (the control point time) for each component.

A control point time (time axis) is set for each control point. The determined control point time (first control point time) which is to be applied to the movement of the components of the treatment machine from their defined axis positions at the first control point to their defined axis positions at the next sequential second control point, is considered to be the control point time of the first control point interval. The determined control point time (second control point time) which is to be applied to the movement of the components of the treatment machine from their defined positions at the second control point to their defined positions at the next sequential third control point, is considered to be the control point time of the second control point interval. The determined control point time (third control point time) which is to be applied to the movement of the components of the treatment machine from their defined positions at the third control point to their defined positions at the next sequential fourth control point, is considered to be the control point time of the third control point interval, and so on.

Following determination of the control point time, the trajectories for each component at each control point are determined at step S305 using the control point time. As mentioned previously each component moves from its first axis position defined in the first control point to a second axis position defined in the second control point. The components do not necessarily move in straight lines. This is because the determined path of the motion is required to take into account the degrees of freedom of the components as well as the positions of the other components of the machine in time, in order to avoid clashes.

Trajectories are then determined for each component of the treatment machine using the trajectories axes and the time axis (the control point time) as an input. Slowing down the speed at which the components move, by virtue of the time axis, changes where each component is in relation to the other components of the treatment machine, when compared to a treatment plan generated using the maximum speeds/accelerations/decelerations possible.

Each component trajectory, determined for each control point, defines a path to be followed by that component as it moves from its axis position at the first control point to its axis position at the next sequential second control point, in the control point time.

According to one embodiment, the trajectories for the couch, gantry and collimator are determined first, since these components tend to move greater distances relative to their maximum velocity, than the other components during a treatment plan and therefore, have a greater effect on the theoretical minimum time and thus the control point time of each control point. The trajectories of the other components (i.e., the leaf positions, delivered dose, and jaw positions) are then determined, so that each component can move from its axis position at the first control point to its axis position at the next sequential second control point, in the control point time.

Since the control point time of each control point sets a time required for a component to move from its axis position at the first control point to its axis position at the second control point and the trajectory sets the path to be followed by the component as it moves from its position at the first control point to its position at the second control point (and thus the distance is known), the speed with which the component should travel can be determined. Although the component may be able to move at a greater speed, the control point time sets an increased amount of time (since it is greater than the minimum theoretical time) for the component to move, thus slowing one or more of the components down from its maximum speed.

The control point time also slows down the acceleration/deceleration of components since is sets an increased amount of time (greater than the minimum time) for the component to move.

At step S306, a smoothed treatment plan is generated comprising the determined control points, control point times and trajectories for each component of the treatment machine.

Although steps S302 to S306 are described as happening sequentially for convenience, these steps may be performed at the same time or iteratively, in order to provide an optimized treatment plan which can deliver the treatment smoothly.

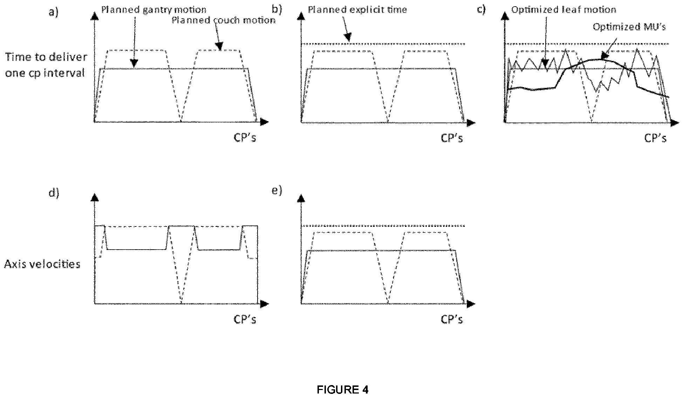

Figures 4A to 4E illustrate schematically axes of a treatment plan. Figure 4A illustrates how much time a gantry and a couch require to travel from previous control point locations to the next control point location, when traveling at their maximum speeds. The gantry and couch are following trajectories that are not synchronised and have not been smoothed in Figure 4A. The trajectories illustrated in Figure 4A represent theoretical minimum times. When the gantry and couch motions are synchronised, there may be accelerations or decelerations of the gantry and/or the couch which result in jerky motions - especially at points when there is change between which of the gantry or the couch requires the longest time and thus dictates the timing of the synchronised motion.

Figure 4B is similar to Figure 4A but includes the specified time axis (planned explicit time), which has been set as greater than the theoretical minimum time as described above.

Figure 4C is similar to Figure 4B but includes the leaf motions and monitor units, which also affect the machine trajectories.

Unlike Figures 4A-4C, where the y-axis represents time, in Figures 4D to 4E the y-axis represents velocity. The unit of velocity may be different for different components of the machine, for example, the velocity of leaf movements are measured in cm/second whereas the velocity of gantry movements are measured in degrees/second. Therefore, in Figures 4D-4E, the velocities have been normalized so that the maximum velocity of each component is the same.

Figure 4D illustrates the velocities of a gantry and a couch when the motions of Figure 4B are synchronised. There is no explicit time axis in Figure 4D. One of the gantry or the couch is always traveling at its maximum speed leading to unnecessary accelerations and decelerations of individual axis and jerky motions.

Figure 4E shows the smoothed velocities of a gantry and a couch when the planned explicit time has been added. The dotted line represents the specified control point time (explicit time) not a velocity. As can be seen in Figure 4E, the gantry and couch have both being slowed down such that their rates of acceleration/decelerations are slower, resulting in a less jerky motion. Note that adding leaf trajectories and MU's don't affect the velocities of gantry and couch motion as long as the minimum time of the leaves and MU delivery are below the planned explicit time.

It is possible for a control point time determined for a second control point to be the same as a control point time determined for a first control point, or any of the other control points. In addition, it is possible for a control point time determined for a second control point to be different from a control point time determined for a first control point, or any of the other control points. Each control point has a control point time set for that control point interval.

As mentioned previously, the control point time is greater than the theoretical minimum time. However, the control point time itself defines a requested time for the components to move from their axis positions at the first control point to their axis position at the second control point. Therefore, if the determined theoretical minimum time is inaccurate or faulty, it is possible for the components to take longer than the control point time to move from their axis positions at the first control point to their axis position at the second control point (in this case the actual delivery of the control point takes more time than the defined control point time). However, the time axis prevents the components from taking less than the control point time to move from their axis positions at the first control point to their axis position at the second control point. For example, the treatment machine may determine that a time greater than the control point time is required for one or more of the control points, this may be as a result of the determinations of trajectories, for example by slowing the components down to the control point time, there is potential for a clash between components, so more time is required to avoid this.

The distance of each trajectory path between the control points is known for each component of the treatment machine, as is the control point time. Accordingly, it is possible to determine a control point speed for each component for each control point. The control point speed is a vector that has a component for each machine axis. The control point speed defines a speed for each of the one or more components of the treatment machine to move in a synchronous manner from their first axis positions at the first control point to their second axis positions at the next sequential second control point. The control point speed is required to be less than the maximum speed of the component.

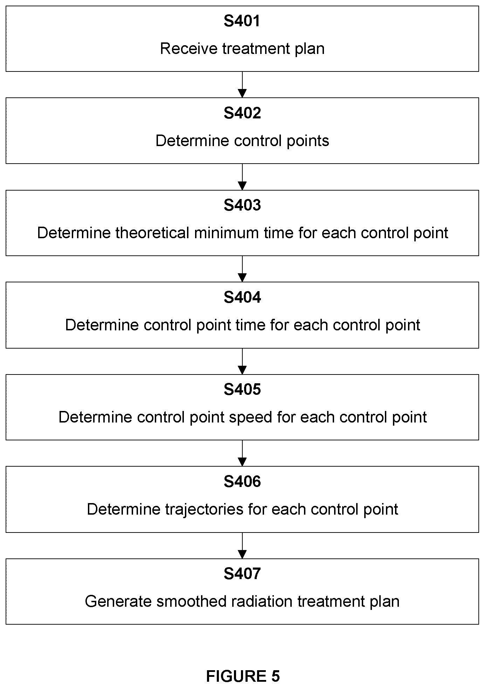

Figure 5 illustrates another method for generating an energy-based treatment plan that can be carried out by the aforementioned control circuit 101. The method of Figure 5 defines a control point speed by determining a minimum speed for one or more machine axis. In this illustrative example, the control circuit 101 is configured to ultimately output a smoothed energy-based treatment plan (such as, for example, a smoothed radiation treatment plan). This energy-based treatment plan typically comprises specified time axis values as well as the specified position axis values for each component and for each control point of a plurality of sequential control points. The control circuit then generates the smoothed treatment plan.

Similar to the method of Figure 3, a predetermined patient treatment plan is input to a treatment system at step S401. The control circuit 101 of the treatment planning system then determines a plurality of control points for the administration of the treatment plan to a patient at step S402. These steps are identical to steps S301 and S302 described above with reference to Figure 3 and therefore further details are not provided here.

At step S403, the control circuit 101 of the treatment system determines a theoretical minimum time required to move the components of the treatment machine from their defined axis positions at the first control point to their defined axis positions at the next sequential control point, i.e., the second control point. At step S404, a control point time is determined for each control point. These steps are identical to steps S303 and S304 described above with reference to Figure 3 and therefore further details are not provided here.

At step S405, a control point speed is adjusted for each control point by setting control point maximum speed for one or more machine axis. Since time, distance and speed are all related by known equation 1 below, it is possible to determine an alternative maximum speed for one or more components, for each control point, based on the determined control point time. A control point maximum speed may be set for each control point and is less than the actual maximum speed of the components of the treatment machine. The maximum control point speed is determined based on the component that takes the longest to move from its axis position at the previous control point to its axis position at the next control. That component will then move at the maximum control point speed. One or more of the other components may then move at a speed less than maximum control point speed.

control point maximum speed = axis motion distance = control point time　　　　　　　　　　Equation 1:

The control point maximum speed is not defined in this manner for all of the components of the treatment machine. Although it is possible to limit a treatment machine so that none of the components move at their maximum speed, this does not prevent the "jerky" movements since limiting the speed of each component merely sets a new "maximum" speed and thus the problem as illustrated in Figure 2 is not addressed.

Instead, the control point maximum speed defines a constant speed for each of the components of the treatment machine to move at. This is particularly advantageous for very heavy components, such as the gantry, which requires more power for accelerations and decelerations than for constant speeds.

The specific control point maximum speed of each component will depend on the distance that component is required to move between the first control point and the second control point. The control point maximum speed is then determined based on the distance and control point time. Consequently, each component of the treatment machine has a different control point speed for each control point. If a component is not required to move during one or more of the control points, then the control point speed is set to zero for that control point.

The control point speed determined for each of the components of the treatment machine to move from their first axis position at the first control point to their second axis position at the second control point is considered to be the control point speed of the first control point interval. The control point speed determined for each of the components of the treatment machine to move from their second axis position at the second control point to their third axis position at the third control point is considered to be the control point speed of the second control point interval. The control point speed determined for each of the components of the treatment machine to move from their third axis position at the third control point to their fourth axis position at the fourth control point is considered to be the control point speed of the third control point interval, and so on.

Following determination of the control point speed, the trajectories for each component of the treatment machine for each control point are determined at step S406 using the control point speed and the trajectories axes as an input.

Finally, at step S407 the determined trajectories and control point speeds are applied to each control point to generate the radiation therapy treatment plan.

Although step S402 to S407 are described as happening sequentially for convenience, these steps may be performed at the same time or iteratively, in order to provide an optimized treatment plan which can deliver the treatment smoothly.

As with the control point time, the control point speed is determined based on a minimum time for the components to move from their axis positions at the first control point to their axis position at the second control point. Therefore, if the control point speed is inaccurately calculated, it is possible for the components to take longer than the control point time, i.e. to move at a speed less than the control point speed, to move from their axis positions at the first control point to their axis position at the second control point - causing the whole treatment to slow down. However, the defined control point maximum speed prevents the components from taking less than the control point time, i.e. prevents the components from moving at a speed greater than the control point speed, to move from their axis positions at the first control point to their axis position at the second control point.

Both the control point time and control point speed are defined in order to limit a mechanical axis of the treatment machine. However, it is also possible to limit a nonmechanical axis of the treatment machine, such as the cumulative meterset weight.

The aim of the treatment machine is to deliver therapeutic energy to a patient in accordance with an energy-based treatment plan. The cumulative meterset weight indicates how much energy/radiation is to be delivered during the control point intervals. The cumulative meterset weight is a cumulative figure (the cumulative meterset weight in the latter control point minus the cumulative meterset weight in the former control point). The units of dose are monitor units (MU). The dose rate indicates how quickly the dose can be delivered and the units are MU/s (monitor units per second). The maximum dose rate is set in the treatment plan. However, the dose rate may be reduced which results in the machine operating more slowly. Therefore, each control point may have a defined energy dose rate (MU/t).

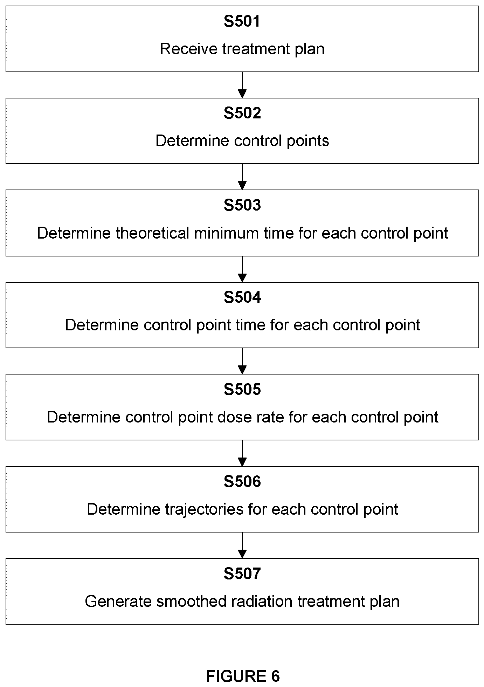

Figure 6 illustrates another method for generating an energy-based treatment plan that can be carried out by the aforementioned control circuit 101. The method of Figure 6 defines a control point dose rate. In this illustrative example the control circuit 101 is configured to ultimately output a smoothed energy-based treatment plan (such as, for example, a smoothed radiation treatment plan). This energy-based treatment plan typically comprises specified time axis values as well as the specified position axis values for each control point of a plurality of sequential control points. The control circuit then generates the smoothed treatment plan.

Similar to the method of Figure 3, a predetermined patient treatment plan is input to a treatment system at step S501. The control circuit 101 of the treatment planning system then determines a plurality of control points for the administration of the treatment plan to a patient at step S502. These steps are identical to those described above with reference to Figure 3 and therefore further details are not provided here.

At step S503, the control circuit 101 of the treatment system determines a theoretical minimum time required to move the components of the treatment machine from their defined axis positions at the first control point to their defined axis positions at the next sequential control point, i.e., the second control point. At step S504, a control point time is determined for each control point. These steps are identical to steps S303 and S304 described above with reference to Figure 3 and therefore further details are not provided here.

Since dose rate is dependent on time, it is possible to determine a control point dose rate for each control point based on the determined control point time. For example, it may be defined in the treatment plan that x doses (MU's) of energy are to be applied as the gantry moves the energy source 115 from the first control point to the second control point. Since the control point time is determined at step S504, the control point dose rate can be determined using equation 2 below.

$$\text{Equation 2: control point dose rate (MU/s)} = \text{dose} \div \text{control point time}$$

Accordingly, at step S505, a control point dose rate is determined for each control point. The control point does rate (first control point does rate) which is to be applied to the movement of the energy source from its defined axis position at the first control point to its defined axis position at the next sequential second control point, is considered to be the control point does rate of the first control point interval. The determined control point does rate (second control point does rate) which is to be applied to the movement of the energy source from its defined axis position at the second control point to its defined axis position at the next sequential third control point, is considered to be the control point dose rate of the second control point interval. The determined control point dose rate (third control point dose rate) which is to be applied to the movement of the energy source from its defined axis position at the third control point to its defined axis position at the next sequential fourth control point, is considered to be the control point dose rate of the third control point interval, and so on.

Following determination of the control point dose rate, the trajectories for each control point are determined at step S506 using the control point dose rate as an input.

Finally, at step S507 the determined trajectories, control point times and control point dose rates are applied to each control point to generate the radiation therapy treatment plan.

Since the dose rate is dependent on the control point time, if the gantry takes longer than the control point time to move the energy source from its axis positions at the first control point to its axis position at the second control point, then the dose rate can be reduced, so that a stable amount of energy is applied to the patient.

Although step S502 to S507 are described as happening sequentially for convenience, these steps may be performed at the same time or iteratively, in order to provide an optimized treatment plan which can deliver the treatment smoothly.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

**Claims**

1. A method of generating a radiation therapy treatment plan, the treatment plan comprising at least two consecutive control points, each control point comprising an axis position for each one or more moveable components of a treatment machine at that control point, the method comprising:

    determining a theoretical minimum time for each control point, the theoretical minimum time comprising a time for all of the one or more components of the treatment machine to move from their first axis position at a first control point to their second axis position at a consecutive second control point, the theoretical minimum time determined based on a maximum speed each of the one or more components of the treatment machine is capable of moving; determining a control point time for each control point, the control point time comprising a time for all of the one or more components of the treatment machine to move from their first axis positions at the first control point to their second axis positions at the second control point, wherein the control point time is greater than the theoretical minimum time; determining trajectories for each of the one or more components of the treatment machine for each control point, the trajectory for each of the one or more components of the treatment machine comprising a path for the component of the treatment machine to move from their first axis position at the first control point to their second axis position at the second control point within the control point time; and applying the determined trajectories and the determined control point times to each control point to generate the radiation therapy treatment plan.

2. The method of claim 1, further comprising:
synchronising the one or more components of the treatment machine to all move from their first axis positions at the first control point to their second axis positions at the second control point and arrive at their second axis positions at the second control point at the control point time.

3. The method of claim 1, further comprising:

determining a time greater than the control point time to move all of the one or more components of the treatment machine from their first axis positions at the first control point to their second axis positions at the second control point, when one or more of the components of the treatment machine requires greater than the control point time to move from their first axis positions at the first control point to their second axis positions at the second control point; and applying the determined trajectories and the determined time greater than the control point time to the first control point to generate the radiation therapy treatment plan.

4. The method of claim 3, further comprising:
synchronising the one or more components of the treatment machine to all move from their first axis positions at the first control point to their second axis positions at the second control point and arrive at their second axis positions at the second control point at the time greater than the control point time.

5. The method of any one of claims 1 to 4, further comprising:

determining a control point speed for each of the one or more components of the treatment machine for each control point, wherein the control point speed for each of the one or more components of the treatment machine is determined using the control point time and a distance between the first axis position of the component at the first control point and the second axis positions of the component at the second control point; and applying the control point speed to each of the one or more components of the treatment machine for each control point to generate the radiation therapy treatment plan.

6. The method of claim 3, further comprising:

determining a control point speed for each of the one or more components of the treatment machine for each control point, wherein the control point speed for each of the one or more components of the treatment machine is determined using the time greater than the control point time and a distance between the first axis position of the component at the first control point and the second axis positions of the component at the second control point; and applying the control point speed to each of the one or more components of the treatment machine for the first control point to generate the radiation therapy treatment plan.

7. The method of any one of claims 1 to 6, wherein:

a control point time determined for a second control point is the same as a control point time determined for a first control point; or a control point time determined for a second control point is different from a control point time determined for a first control point.

8. The method of any one of claims 1 to 7, wherein the trajectory for one or more components of the treatment machine comprises no trajectory so that the one or more components of the treatment machine remain at the first axis position at the second control point.

9. The method of any one of claims 1 to 10, wherein the first axis position at the first control point of each of the one or more components may be the same as or different from the first axis position at the first control point of the other one or more components.

10. An apparatus to facilitate generation of a radiation treatment plan, the treatment plan comprising at least two consecutive control points, each control point comprising an axis position for each one or more moveable components of a treatment machine at that control point, the apparatus comprising:

a control circuit (101) configured to:

determine a theoretical minimum time for each control point, the theoretical minimum time comprising a time for all of the one or more components of the treatment machine to move from their first axis position at a first control point to their second axis position at a consecutive second control point, the theoretical minimum time determined based on a maximum speed each of the one or more components of the treatment machine is capable of moving;

determine a control point time for each control point, the control point time comprising a time for all of the one or more components of the treatment machine to move from their first axis positions at the first control point to their second axis positions at the second control point, wherein the control point time is greater than the theoretical minimum time;

determine trajectories for each of the one or more components of the treatment machine for each control point, the trajectory for each of the one or more components of the treatment machine comprising a path for the component of the treatment machine to move from their first axis position at the first control point to their second axis position at the second control point within the control point time; and

apply the determined trajectories and the determined control point times to each control point to generate the radiation therapy treatment plan.

11. The apparatus of claim 10, wherein the control circuit (101) is further configured to:
synchronise the one or more components of the treatment machine to all move from their first axis positions at the first control point to their second axis positions at the second control point and arrive at their second axis positions at the second control point at the control point time.

12. The apparatus of claim 10 or claim 11, wherein the control circuit (101) is further configured to:

determine a time greater than the control point time to move all of the one or more components of the treatment machine from their first axis positions at the first control point to their second axis positions at the second control point, when one or more of the components of the treatment machine requires greater than the control point time to move from their first axis positions at the first control point to their second axis positions at the second control point; and

apply the determined trajectories and the determined time greater than the control point time to the first control point to generate the radiation therapy treatment plan.

13. The apparatus of claim 12, wherein the control circuit (101) is further configured to:
synchronise the one or more components of the treatment machine to all move from their first axis positions at the first control point to their second axis positions at the second control point and arrive at their second axis positions at the second control point at the time greater than the control point time.

14. The apparatus of any one of claims 10 to 13, wherein the control circuit (101) is further configured to:

determine a control point speed for each of the one or more components of the treatment machine for each control point, wherein the control point speed for each of the one or more components of the treatment machine is determined using the control point time and a distance between the first axis position of the component at the first control point and the second axis positions of the component at the second control point; and

apply the determined control point speed to each of the one or more components of the treatment machine for each control point to generate the radiation therapy treatment plan.

**15.** The apparatus of claims 12 to 14, wherein the control circuit (101) is further configured to:

determine a control point speed for each of the one or more components of the treatment machine for each control point, wherein the control point speed for each of the one or more components of the treatment machine is determined using the time greater than the control point time and a distance between the first axis position of the component at the first control point and the second axis positions of the component at the second control point; and

apply the determined control point speed to each of the one or more components of the treatment machine for the first control point to generate the radiation therapy treatment plan.

FIGURE 1

FIGURE 2

**S301**

Receive treatment plan

**S302**

Determine control points

**S303**

Determine theoretical minimum time for each control point

**S304**

Determine control point time for each control point

**S305**

Determine trajectories for each control point

**S306**

Generate smoothed radiation treatment plan

FIGURE 3

FIGURE 4

```
┌─────────────────────────────────────────────────────┐
│                      S401                           │
│                                                     │
│              Receive treatment plan                 │
└─────────────────────────────────────────────────────┘
                          │
                          ▼
┌─────────────────────────────────────────────────────┐
│                      S402                           │
│                                                     │
│             Determine control points                │
└─────────────────────────────────────────────────────┘
                          │
                          ▼
┌─────────────────────────────────────────────────────┐
│                      S403                           │
│                                                     │
│   Determine theoretical minimum time for each control point │
└─────────────────────────────────────────────────────┘
                          │
                          ▼
┌─────────────────────────────────────────────────────┐
│                      S404                           │
│                                                     │
│      Determine control point time for each control point │
└─────────────────────────────────────────────────────┘
                          │
                          ▼
┌─────────────────────────────────────────────────────┐
│                      S405                           │
│                                                     │
│      Determine control point speed for each control point │
└─────────────────────────────────────────────────────┘
                          │
                          ▼
┌─────────────────────────────────────────────────────┐
│                      S406                           │
│                                                     │
│       Determine trajectories for each control point │
└─────────────────────────────────────────────────────┘
                          │
                          ▼
┌─────────────────────────────────────────────────────┐
│                      S407                           │
│                                                     │
│       Generate smoothed radiation treatment plan    │
└─────────────────────────────────────────────────────┘
```

FIGURE 5

**S501**

Receive treatment plan

**S502**

Determine control points

**S503**

Determine theoretical minimum time for each control point

**S504**

Determine control point time for each control point

**S505**

Determine control point dose rate for each control point

**S506**

Determine trajectories for each control point

**S507**

Generate smoothed radiation treatment plan

FIGURE 6

Europäisches
Patentamt
European
Patent Office
Office européen
des brevets

# EUROPEAN SEARCH REPORT

**Application Number**

EP 24 22 0678

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/069278 A1 (BOSE SUPRATIK [US] ET AL) 5 March 2020 (2020-03-05) * paragraphs [0003], [0039], [0045], [0061] - [0069], [0098] - [0111]; figure 5 * | 1-15 | INV. A61N5/10 |
| | ----- | | |
| A | US 7 961 843 B2 (ELEKTA AB [SE]) 14 June 2011 (2011-06-14) * claim 1 * | 1-15 | |
| | ----- | | |

**TECHNICAL FIELDS SEARCHED (IPC)**

A61N

The present search report has been drawn up for all claims

| Place of search | Date of completion of the search | Examiner |
|---|---|---|
| Munich | 9 May 2025 | Kajzar, Anna |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 22 0678

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2025

| Patent document cited in search report | | Publication date | Patent family member(s) | | Publication date |
|---|---|---|---|---|---|
| US 2020069278 | A1 | 05-03-2020 | CN | 107198832 A | 26-09-2017 |
| | | | EP | 3257553 A1 | 20-12-2017 |
| | | | EP | 3750599 A1 | 16-12-2020 |
| | | | US | 2017354393 A1 | 14-12-2017 |
| | | | US | 2020069278 A1 | 05-03-2020 |
| | | | US | 2020383657 A1 | 10-12-2020 |
| | | | US | 2022167940 A1 | 02-06-2022 |
| US 7961843 | B2 | 14-06-2011 | AT | E439164 T1 | 15-08-2009 |
| | | | CA | 2650421 A1 | 08-11-2007 |
| | | | CN | 101472649 A | 01-07-2009 |
| | | | EP | 2010287 A1 | 07-01-2009 |
| | | | JP | 5031823 B2 | 26-09-2012 |
| | | | JP | 2009534135 A | 24-09-2009 |
| | | | US | 2009213991 A1 | 27-08-2009 |
| | | | WO | 2007124760 A1 | 08-11-2007 |

EPO FORM P0459

For more details about this annex : see Official Journal of the European Patent Office, No. 12/82